# DEMANDE DE BREVET EUROPEEN

(11) **EP 1 964 481 A1**
(43) Date de publication de la demande: **03.09.2008**
(21) Numéro de dépôt: 08101381.5
(22) Date de dépôt: 07.02.2008
(51) Int. Cl.: A23L 1/30, A61K 36/886, A61K 35/74

(54) **Complément alimentaire comprenant de la spiruline et un produit à base de plante du genre Aloe, et son utilisation cosmétique**

(30) Priorité: 14.02.2007 MA 29680; 14.05.2007 FR 0755046
(71) Demandeur: Alleon, Philippe, Inezgane (MA)
(72) Inventeur: Alleon, Philippe, Inezgane (MA)
(74) Mandataire: Domenego, Bertrand

(57) **Abrégé**

L'invention concerne une composition comprenant de la spiruline ou un produit dérivé de la spiruline et un produit à base de plante du genre *Aloe;* son utilisation pour fabriquer un complément alimentaire et ledit complément alimentaire en résultant.

## Description

L'invention concerne un complément alimentaire pour renforcer les défenses naturelles.

Le genre *Aloe* (division des Magnoliophyta, classe des Liliopsida, ordre des Asparagales, famille des Asphodelaceae) comprend environ 400 espèces de plantes succulentes à fleurs communément appelées aloès dont la plus connue est sûrement l'*Aloe vera.* La plupart des aloès présentent une rosette de feuilles larges, épaisses et piquantes en forme de lance. Ils paraissent souvent sans tige, la rosette poussant directement sur le sol.

Il est connu d'utiliser des produits dérivés de l'*Aloe vera* dans des préparations cosmétiques.

Les spirulines sont un genre (*Arthrospira*) de cyanobactéries - anciennement appelées cyanophycées ou « algues bleues » - des eaux chaudes peu profondes et saumâtres de la ceinture intertropicale. Bien que communément appelée "spiruline" ("spirulina" en anglais), cette cyanobactérie appartient au genre *Arthrospira.* Le genre *Spirulina* existe, mais s'applique à d'autres cyanobactéries, assez éloignées du point de vue taxonomique.

Il s'agit d'algues microscopiques photosynthétiques qui poussent naturellement dans les eaux alcalines de certains lacs de la zone intertropicale.

En raison de leur richesse exceptionnelle en protéines, les spirulines sont cultivées dans de nombreux pays, depuis les années 1970 : Afrique, Antilles, Chine, États-Unis, Hawaii, Inde, Madagascar, Mexique, Équateur, Thaïlande... Depuis peu, on les cultive en France, dans les Cévennes et dans le Var.

Les spirulines permettent de lutter contre la malnutrition, la dénutrition et les carences protéiques, telles le kwashiorkor. Elles sont une source de fer hautement assimilable. Riche en molécules anti-oxydantes (acide gamma-linolénique, phycocyanine, tocophérol, carotène, sélénium et zinc), elles sont également utilisées en cosmétologie.

L'un des atouts des spirulines est de croître dans des eaux faiblement à fortement salées, et alcalines, c'est-à-dire dans des conditions où la croissance des autres microorganismes est limitée. Il en résulte que l'on peut obtenir assez facilement des cultures homogènes non contaminées, sur des périodes relativement courtes.

Un autre avantage des spirulines est lié au bon rendement des cultures. Dans des bassins aquatiques de quelques décimètres de profondeur exposés au soleil, dans une eau basique (pH proche de 10) et maintenue à une température comprise entre 25 et 35°C, la production atteint 10 g/j/m². Après récolte, filtration, égouttage, lavage, puis séchage, on obtient une fine poudre verte.

Les spirulines ont fait l'objet d'études toxicologiques exhaustives qui ont démontré sa parfaite innocuité.

Au delà de l'intérêt que présentent les cultures de spirulines pour venir en aide aux populations touchées par la malnutrition, les spirulines connaissent un succès croissant dans les pays industrialisés auprès des sportifs et des nutritionnistes qui trouvent dans ce produit naturel une source exceptionnelle de principes actifs.

On citera en particulier des teneurs intéressantes en :
- fer particulièrement bio-disponible, donc directement absorbable,
- vitamine B9, ou acide folique, qui participe à la fixation du fer et à l'anabolisme musculaire,
- acides aminés ramifiés, Leucine, Isoleucine, Valine, particulièrement recherchés pour la prise de masse musculaire,
- vitamines B1, B6 et B12, dont le rôle est essentiel tant pour la préparation que pour la récupération à l'effort,
- vitamines liposolubles D, E, A et surtout provitamine A,
- bêta carotène qui élimine les risques de surdosage,
- métaux (dans des proportions non toxiques): Sélénium, Cuivre, Magnésium, Manganèse,
- enzymes et co-enzymes qui interviennent dans les réactions métaboliques, dont la Super Oxyde Dismutase (SOD) qui est un puissant antioxydant,
- acides gras polyinsaturés oméga 3 et oméga 6, plus particulièrement les acides linolénique et di-homo-gamma linolénique, dont l'apport est essentiel pour l'organisme; et
- phycocyanine, principal pigment photosynthétique des spirulines, aux propriétés également anti-oxydantes.

Parmi ces principes actifs la phycocyanine, la SOD et les acides gras polyinsaturés di-homo-gamma linolénique et gamma linolénique, sont les plus recherchés. La phycocyanine est une phycobiliprotéine de 332 kd proche dans sa structure des pigments biliaires humains, qui sont connus pour leurs propriétés anti-inflammatoires. Des études ont souligné que la phycocyanine permettait chez l'animal, d'inhiber la formation des leucotriènes B4, qui sont des métabolites eicosanoïdes hautement inflammatoires, ainsi que l'activité de la cyclo-oxygénase-2 (COX-2) et de la lipoxygénase, des enzymes associées aux processus inflammatoires [Romay C. et al., Inflamm. Res., 1998, 47(1):36-41].

La demande internationale WO99/15688 divulgue une méthode pour cultiver des spirulines afin de produire une biomasse particulièrement riche en omega 6, acides gras polyinsaturés, en particulier acide gamma-linoléique, et/ou en sulfolipides.

Le Demandeur a maintenant découvert, de manière surprenante, que l'association de spiruline et d'un produit à base de plante du genre *Aloe* permet d'obtenir une activité très supérieure à celle de la spiruline seule, en particulier pour renforcer les défenses naturelles de l'organisme ou lutter contre la fatigue physique.

Ainsi, selon un premier aspect, la présente invention a pour objet une composition comprenant de la spiruline ou un produit dérivé de la spiruline et un produit à base de plante du genre *Aloe.*

Dans le cadre de la présente invention, on entend par "produit à base de plante du genre *Aloe"* un produit dérivé d'une plante du genre *Aloe,* par exemple un jus, une pulpe, un extrait, une huile, un lyophilisat, un produit de déshydratation, un produit de zéodratation ou un mélange de ceux-ci obtenu(e) à partir d'un organe, d'un liquide ou d'un broyat provenant directement d'une plante du genre *Aloe.* De préférence, ce produit dérivé est obtenu à partir des feuilles.

Dans le cadre de la présente invention, on entend de préférence par « plante du genre *Aloe »* l'*Aloe vera* ou l'*Aloe arborescens,* de manière particulièrement préférée *l'Aloe arborescens.*

Par spiruline, on désigne une algue bleue-verte appartenant au phyllum Cyanophyta, classe: Cyanophyceae, ordre : Nostocales, famille: Oscillatoriaceae, genre: Arthrospira.

Dans le cadre de la présente invention, on entend par "produit dérivé de la spiruline" une culture de spirulines séparée de son milieu de culture, éventuellement lavée, éventuellement égouttée, et/ou éventuellement séchée, ou un extrait, un lyophilisat, un produit de déshydratation, un produit de zéodratation ou un mélange de ceux-ci obtenu à partir d'une culture de spiruline.

Avantageusement, la spiruline ou le produit dérivé de la spiruline est présent dans la composition selon l'invention à raison d'environ 10% à environ 80%, de préférence environ 20% à environ 60%, en particulier environ 50% en poids de la composition.
Avantageusement encore, le produit à base de plante du genre *Aloe* est présent à raison d'environ 10% à environ 80%, de préférence d'environ 20% à environ 60%, en particulier environ 50% en poids de la composition.
Les compositions selon l'invention peuvent comprendre, en tant que composant supplémentaire, un extrait de noni (Morinda citrifolia).

Un procédé de préparation d'une composition selon l'invention est un objet supplémentaire de la présente invention.
Dans un tel procédé de préparation, on mélange de la spiruline ou un produit dérivé de la spiruline et un produit à base de plante du genre *Aloe.*

Un autre objet de la présente invention est l'utilisation des compositions selon l'invention pour préparer des compléments alimentaires. De tels compléments alimentaires font également partie de l'invention.

De façon à assurer un effet optimal au sein du complément alimentaire dans lequel elle est incorporée, la composition selon l'invention est généralement mise en oeuvre dans ledit complément à raison de 1 à 100% en poids, de préférence à raison d'environ 5% à environ 95% en poids, de manière particulièrement préférée à raison d'environ 60% à environ 90% en poids.
Les compléments alimentaires selon l'invention peuvent comprendre, en plus des compositions selon l'invention, un ou plusieurs véhicules acceptables sur le plan alimentaire.

Par « véhicule acceptable sur le plan alimentaire», on entend un véhicule adapté pour une utilisation par voie orale, sans toxicité ou réponse allergique indue et similaire, et proportionné à un rapport avantage/risque raisonnable.
Les compléments alimentaires selon l'invention peuvent comprendre, en plus des compositions selon l'invention, un ou plusieurs autres additifs acceptables sur le plan alimentaire, choisis de préférence dans le groupe constitué de colorants, agents de conservation, antioxydants, agents de texture, amidons modifiés, édulcorants, exhausteurs de goût, acidifiants, émulsifiants, épaississants, stabilisants et gélifiants.

De tels compléments alimentaires selon l'invention se présentent généralement sous forme de comprimé, tablette, barre, boisson, gélule, poudre ou granulés.
Il est des compétences de l'homme du métier de déterminer la nature du ou des composant(s) supplémentaire(s) à mettre en oeuvre dans les compléments alimentaires de l'invention compte tenu du type de complément qu'il souhaite obtenir.

Un complément alimentaire selon l'invention peut être préparé selon n'importe quelle méthode connue de l'homme du métier. Une telle méthode peut comprendre la mise en forme dudit complément sous forme de comprimés, tablettes, etc, en particulier par extrusion.

De préférence, le complément alimentaire de la présente invention est destiné à une utilisation chez l'être humain, les animaux de compagnie, en particulier les chiens ou les chats, ou les animaux d'élevage.

Un autre objet de l'invention est l'utilisation d'un complément alimentaire selon l'invention pour renforcer les défenses naturelles de l'organisme.
Un autre objet de l'invention est l'utilisation d'un complément alimentaire selon l'invention pour augmenter l'endurance et la résistance de l'organisme.
Un autre objet de l'invention est l'utilisation d'un complément alimentaire selon l'invention pour lutter contre la fatigue physique.
Un autre objet de l'invention est l'utilisation d'un complément alimentaire selon l'invention pour redonner de l'éclat à la peau, aux ongles et/ou aux cheveux.
Un autre objet de l'invention est l'utilisation d'un complément alimentaire selon l'invention pour complémenter un régime minceur.

De préférence, le complément alimentaire selon l'invention est utilisé chez les malnutris, les convalescents, les anorexiques, les femmes enceintes ou allaitantes, les sportifs et les personnes âgées.

Les exemples suivants illustrent l'invention sans en limiter la portée.

### EXEMPLE 1 : Préparation d'une composition selon l'invention à partir de matières fraîches

On mélange une culture de spiruline récoltée, lavée et égouttée avec un jus de feuille d'*Aloe arborescens* obtenu par broyage de feuilles, selon les proportions suivantes :

| | |
|---|---|
| Spiruline | 50% |
| *Aloe arborescens* | 50% |

Le produit obtenu est un liquide sirupeux.

### EXEMPLE 2 : Préparation d'une composition selon l'invention à partir de matières sèches

On mélange une culture de spiruline récoltée, lavée, égouttée et séchée avec un broyat de feuilles *d'Aloe arborescens* séché, selon les proportions suivantes :

| | |
|---|---|
| Spiruline | 80% |
| *Aloe arborescens* | 20% |

Le produit obtenu est une poudre.

## Revendications

1. Composition comprenant de la spiruline ou un produit dérivé de la spiruline et un produit à base de plante du genre *Aloe.*

2. Composition selon la revendication 1, **caractérisée en ce que** ledit produit à base de plante du genre *Aloe* est un jus de feuille d'une plante du genre *Aloe.*

3. Composition selon l'une quelconque des revendications 1 à 2, **caractérisée en ce que** ladite plante du genre *Aloe* est l'*Aloe arborescens.*

4. Composition selon l'une quelconque des revendications 1 à 2, comprenant en outre un extrait de noni (Morinda citrifolia).

5. Composition selon l'une quelconque des revendications 1 à 4, **caractérisée en ce que** la spiruline ou le produit dérivé de la spiruline est présent à raison d'environ 10% à environ 80% en poids de ladite composition

6. Composition selon l'une quelconque des revendications 1 à 5, **caractérisée en ce que** ledit produit à base de plante du genre *Aloe* est présent à raison d'environ 10 à environ 80% en poids de ladite composition.

7. Utilisation d'une composition selon l'une quelconque des revendications 1 à 6 pour la préparation d'un complément alimentaire.

8. Complément alimentaire comprenant une composition selon l'une quelconque des revendications 1 à 6.

9. Utilisation d'un complément alimentaire selon la revendication 8 pour renforcer les défenses naturelles de l'organisme.
